# FASCICULE DE BREVET EUROPEEN

(11) **EP 1 127 036 B1**
(45) Date de publication et mention de la délivrance du brevet: **11.01.2006**
(21) Numéro de dépôt: 99954061.0
(22) Date de dépôt: 04.11.1999
(51) Int. Cl.: C07B 45/00, C07C 303/38, C07C 311/09

(54) **PROCEDE ET REACTIF DE SULFONYLATION UTILES POUR LA SYNTHESE DE SULFANILIDE PERHALOGENE**
ZUR SULFONIERUNG VON PERHALOGENIERTEN SULFANILID VERWENDBARE VERFAHREN UND REAGENZ ZUR SULFONIERUNG
SULPHONYLATION METHOD AND REAGENT USEFUL FOR PERHALOGENATED SULPHANILIDE SYNTHESIS

(30) Priorité: 04.11.1998 FR 9813863; 06.10.1999 FR 9912453
(43) Date de publication de la demande: 29.08.2001
(73) Titulaire: RHODIA CHIMIE, 92512 Boulogne Billancourt Cedex (FR)
(72) Inventeur: DESMURS, Jean-Roger, Communay, 69360 Saint-Symphorien d'Ozon (FR); MILLET, André, F-69720 Saint-Laurent de Mure (FR); PEVERE, Virginie, F-69008 Lyon (FR)
(74) Mandataire: Esson, Jean-Pierre
(86) Numéro de dépôt international: PCT/FR1999/002697
(87) Numéro de publication internationale: WO 2000/026161

(56) Documents cités:
- EP-A- 0 778 268
- FR-A- 2 217 322
- J. BURDON, ET AL.: "Fluorinated sulphonic acids. Part I. Perfluoro-methane-, -octane-, and -decane-sulphonic acids and their simple derivatives" JOURNAL OF THE CHEMICAL SOCIETY, no. 6, juin 1957 (1957-06), pages 2574-2578, XP000563885 ROYAL SOCIETY OF CHEMISTRY, LETCHWORTH, GB
- A.P. KOZIKOWSKI, ET AL.: "The intramolecular nitrile oxide cycloaddition approach to the mitomycins" JOURNAL OF THE CHEMICAL SOCIETY, CHEMICAL COMMUNICATIONS, no. 3, 1 février 1988 (1988-02-01), pages 198-200, XP002109015 ROYAL SOCIETY OF CHEMISTRY, LETCHWORTH, GB ISSN: 0022-4936
- M. KIZIL, ET AL.: "Studies of the tetrathiafulvalene mediated radical-polar crossover reaction directed toward the total synthesis of alkaloid natural products" TETRAHEDRON LETTERS, vol. 37, no. 14, 1 avril 1996 (1996-04-01), pages 2511-2514, XP004029981 ELSEVIER SCIENCE PUBLISHERS, AMSTERDAM, NL ISSN: 0040-4039

## Description

La présente invention a pour objet un procédé de sulfonylation de divers nucléophiles, notamment azotés. Elle concerne plus particulièrement la sulfonylation des amines et plus particulièrement des anilines lato sensu, c'est-à-dire les amines liées à un noyau aromatique.

La réaction vise aussi un réactif de perhalogénoalcanesulfonylation. Ainsi, la présente invention concerne plus particulièrement une réaction de sulfonylation d'une amine porteuse d'un radical électro-attracteur surtout quand ses fonctions aminées sont rendues molles, par exemple par la présence d'un radical arylique (l'amine entrant alors dans la sous-catégorie des anilines).

La présente invention vise aussi la perhalogénosulfonylation de l'amine très particulière que constitue l'ammoniac, pour conduire soit à l'amide soit à l'imide.

La synthèse de ces dérivés de type sulfonamide est souvent difficile, surtout lorsque l'on part d'halogénure de sulfonyle. La demande de brevet n°FR 2-217-322 fait appel aux fluorures de sulfonyle en l'absence de catalyseur; mais ces techniques sont difficilement transposables pour les chlorures et les bromures. Le plus souvent, les réactions directes sont des échecs et ce en particulier avec les chlorures de sulfonyle, surtout quand la partie organique des sulfonyles est très électro-attractrice comme notamment dans le cas où l'atome porteur du soufre de la fonction sulfonyle est perhalogéné et plus particulièrement quand il est perfluoré. L'explication de ces échecs semble liée au caractère oxydant des halogénures de sulfonyle, notamment de trifluorométhane sulfonyle qui, comme le chlorure de sulfuryle, est un oxydant efficace.

C'est pourquoi un des buts de la présente invention est de fournir un procédé qui permette d'obtenir des sulfonamides du type précédent en utilisant des halogénures de sulfonyle, en particulier quand ces halogénures sont des halogénures lourds (c'est-à-dire des halogénures correspondant à un halogène de nombre atomique au moins égal à celui du chlore).

On préfère utiliser pour des raisons économiques, mais aussi techniques les chlorures de sulfonyle. Il convient en effet de signaler d'une part que l'iodure est difficile d'accès, et même parfois que son existence est douteuse, et d'autre part que le bromure est relativement instable. La technique a également été transposée pour la synthèse des perfluorosulfonimides, en une ou deux étapes.

Ces buts et d'autres qui apparaîtront par la suite sont atteints par un procédé de sulfonylation comprenant une étape de mise en contact d'un nucléophile dont l'atome nucléophile est avantageusement un azote, avec un réactif comportant pour addition successive ou simultanée :
- un halogénure lourd c'est à dire, dont le nombre atomique est au moins egal à celui du chlore de sulfonyle, avantageusement chlorure de sulfonyle
   et
- une base organique comportant un azote trisubstitué et présentant un enchaînement de formule > N-[C = C]ₙ-C = N- avec n égal à zéro ou un entier choisi dans l'intervalle fermé 1 à 4, avantageusement de 1 à 3, de préférence de 1 à 2.
et par le fait que la partie organique dudit sulfonyle est perhalogénée, avantageusement perfluorée, sur le carbone porté par le soufre

Il est souhaitable que ledit azote trisubstitué de ladite base organique présente un nombre donneur supérieur à celui de la pyridine.

Pour la définition de l'indice donneur ("donor number") on peut se référer à l'ouvrage de Christian Reincardt, *Solvents and solvents effects in organic chemistry,* p. 19 (1988), ouvrage où l'on'trouve comme définition le négatif de l'enthalpie (-ΔH exprimé en kilocalorie/mol) de l'interaction entre le solvant et le pentachlorure d'antimoine dans une solution diluée de dichloro-méthane.

Quoique moins satisfaisant que le chlorure, le bromure de sulfonyle peut également être utilisé.

La présente invention est particulièrement intéressante pour les nucléophiles dont l'acide associé présente un pKa au plus égal à environ 7, avantageusement à 6, de préférence à 5, plus préférentiellement à 4. Elle est également intéressante pour les nucléophiles oxydables et plus généralement lorsque l'on désire utiliser un réactif oxydable.

En effet, ces nucléophiles sont en général particulièrement difficiles à sulfonyler. En particulier, l'invention est intéressante pour les nucléophiles dont l'atome nucléophile, souvent un azote, est relié à un aryle ou un groupe électro-attracteur ou, plus généralement, à un ensemble électro-attracteur de substituants, c'est à dire tel que la somme de leurs constantes de Hammett σₚ soit au moins égale à 0,2, avantageusement à 0,3, de préférence à 0,5.

Ce groupe électro-attracteur peut notamment être choisi parmi les aryles, avantageusement appauvris en électrons, et les restes d'acides, avantageusement oxygénés, restes tels que phosphoryles, phosphonyles, acyles, et surtout sulfonyles.

Ladite base organique comportant un azote trisubstitué saturé, présentant une résonance avec une liaison π, peut être utilisée soit comme base, soit comme catalyseur de la réaction.

En effet, la réaction de sulfonylation dégage un acide halohydrique qui souvent salifie le nucléophile et rend alors le nucléophile plus ou moins inerte. En outre, souventes fois, le produit de sulfonylation est un acide plus fort que l'acide conjugué de la base ; dans ce cas il convient aussi de prévoir la neutralisation de cet acide.

Aussi est-il souhaitable d'ajouter des bases (en quantité et en nature) qui permettront de libérer le nucléophile des différents acides présents dans le milieu réactionnel de manière qu'il joue pleinement son rôle de nucléophile.

Ladite base organique comportant un azote trisubstitué saturé, présentant une résonance avec une liaison n est avantageusement telle que ledit atome métalloïde est un atome trivalent de la colonne VB, lequel est de préférence un atome trisubstitué formant une base tertiaire.

Selon une mise en oeuvre particulièrement avantageuse de la présente invention, ladite liaison π reliant deux atomes est la liaison π d'une fonction imine.

Il est préférable que cette fonction imine soit disposée de telle manière que les atomes d'azote et dudit métalloïde soient le plus éloignés possible, en d'autres termes et par exemple, que l'azote de la fonction imine soit celui des deux atomes reliés par la liaison n qui est le plus éloigné de l'atome trivalent de la colonne V. Ce qui vient d'être dit à propos de la fonction imine est général pour tous les atomes de la colonne VB reliés par la liaison π, dans le cas où la liaison π comporte un atome de carbone et un atome de la colonne V.

Selon la présente invention, il est préféré que la base organique comportant un atome trivalent de la colonne VB dont le doublet est conjugué à une liaison π présente un enchaînement ou un squelette, de formule > N-[C = C]ₙ-C = N-, avec n égal à zéro ou un entier choisi dans l'intervalle fermé (c'est-à-dire comprenant les bornes) 1 à 4, avantageusement de 1 à 3, de préférence de 1 à 2. De préférence l'enchaînement précédent répond à la formule >N-[C(R₁) = C(R₂)]n-C(R₃) = N- avec n égal à zéro ou un entier choisi dans l'intervalle fermé (c'est-à-dire comprenant les bornes) 1 à 4, avantageusement de 1 à 3, de préférence de 1 à 2 et où R₁, R₂ et R₃ semblables ou différents, sont choisis parmi les dérivés hydrocarbonés, avantageusement alcoyles d'au plus 4 atomes de carbone et l'hydrogène. Avantageusement, selon le procédé, ledit atome trivalent de la colonne VB forme ou constitue une amine tertiaire.

Plus précisément, il est souhaitable que ladite base organique comportant un atome trivalent de la colonne VB dont le doublet est conjugué à une liaison π constitue une molécule de formule suivante (R₅)(R₄)N-[C(R₁) = C(R₂)]ₙ-C = N-R₆, avec n égal à zéro ou un entier choisi dans l'intervalle fermé (c'est-à-dire comprenant les bornes) 1 à 4, avantageusement de 1 à 3, de préférence de 1 à 2 et où R₁, R₂ et R₆ semblables ou différents, sont choisis parmi les groupes hydrocarbonés, avantageusement alcoyles d'au plus 4 atomes de carbone et l'hydrogène et où R₄ et R₅, semblables ou différents, sont choisis parmi les groupes hydrocarbonés, avantageusement alcoyles d'au plus 4 atomes de carbone, un ou deux des substituants R₁, R₂, R₃, R₄, R₅ et R₆, pouvant être reliés à d'autre(s) substituant(s) restant pour former un ou plusieurs cycles.

L'effet catalytique constaté est particulièrement marqué quand ladite liaison π reliant deux atomes est intracyclique, surtout quand elle est intracyclique dans un cycle aromatique. C'est notamment le cas des cycles pyridiniques et des cycles qui en sont dérivés comme la quinoléine ou l'isoquinoléine. Ainsi les noyaux pyridiniques enrichis par la présence d'un ou plusieurs atomes métalloïdes, notamment quand la somme des R (voir supra) est au plus égal à - 1,5, avantageusement à - 2, constitue des catalyseurs particulièrement satisfaisants.

Plus spécifiquement la base organique comportant un atome métalloïde saturé, présentant une résonance avec une liaison π peut être avantageusement choisie parmi les dialcoylaminopyridines, notamment en position para- ou ortho-(c'est-à-dire en position 2 ou 4 de la pyridine) ; la DBU (diazabicycloundécène) donne également un résultat intéressant.

Quoique la présente invention puisse être utilisée pour former des sulfonimides usuels, cette réaction est particulièrement intéressante dans le cas de la formation d'une fonction amide ou imide à partir d'un substrat nucléophile, notamment constitué par une aniline et ce, plus particulièrement, lorsque cette aniline est reliée à un noyau aromatique appauvri en électrons.

Cet appauvrissement peut être corrélé à l'introduction d'un hétéroatome dans le cycle (cas des cycles à 6 chaînons) ou bien par la présence de substituants sur le noyau porteur de la fonction aniline à sulfonyler de substituant(s) globalement électro-attracteur(s).

Dans le cas d'un appauvrissement électronique d'un cycle à 6 chaînons par l'introduction d'un hétéroatome, il convient de signaler que le ou, plus précisément, les substrats peuvent être autocatalytiques, c'est-à-dire qu'ils peuvent ne pas nécessiter la présence d'un catalyseur, souvent d'une amine, selon la présente invention.

En ce qui concerne l'appauvrissement par les substituants, on peut donner à titre indicatif que l'invention est particulièrement bien adaptée pour le traitement des arylamines dans laquelle les substituants, hors la fonction nucléophile à sulfonyler, du noyau porteur de l'atome nucléophile sont tels que la somme de leurs constantes de Hammett σₚ soit au moins égale à 0,14, avantageusement à 0,20, de préférence à 0,30.

Lorsque cette somme de constantes de Hammett atteint des valeurs supérieures à 1, la réaction devient particulièrement paresseuse, aussi est-il préférable que la somme des constantes de Hammett du cycle porteur de la fonction amine soit au plus égale à 1, de préférence au plus égale à 0,9, plus préférentiellement au plus égale à 0,7.

Lorsque la base organique comportant un azote trisubstitué, est utilisée comme catalyseur (c'est-à-dire qu'elle est utilisée en quantité sous-stoechiométrique, plus généralement en quantité comprise entre 1‰ et 1/5 de la QS [quantité stoechiométrique] nécessaire à la neutralisation des acides dégagés par la réaction, avantageusement entre 1/100 et 1/10 de QS), il convient alors de prévoir une autre base de manière que la réaction vis-à-vis du substrat nucléophilé soit la plus complète possible.

Dans ce cas, le réactif utilisé comporte en outre pour addition successive ou simultanée une base organique de préférence non alcoylable. On peut en particulier choisir comme base organique non alcoylable les dialcoylphosphines encombrées, les trialcoylphosphines, les hydroxydes de phosphonium quaternaire, les dialcoylamines encombrées, les trialcoylamines, les hydroxydes d'ammonium quaternaire. La notion d'encombrement des dialcoylphosphines ou dialcoylamines encombrées de manière qu'elles ne soient pas alcoylables est bien connue de l'homme du métier.

Dans un grand nombre de cas, et notamment lorsqu'on utilise des solvants, il est préférable que ladite base non alcoylable soit liposoluble et présente au moins une solubilité dans le benzène significative (symbole "s" dans le "Handbook of Chemistry and Physics"), avantageusement (symbole "v" dans le "Handbook of Chemistry and Physics") élevée.

Comme on l'a vu précédemment, il est le plus souvent souhaitable de réaliser la mise en contact dans un solvant organique. Ce solvant est avantageusement peu polaire et, de préférence, peu miscible à l'eau. Plus particulièrernent, il est souhaitable qu'au plus 10% en masse, avantageusement au plus 5%, de préférence au plus 2% en masse, soit miscible à l'eau.

Les quantités de base à ajouter, la quantité de base non alcoylable mise en jeu au cours de la réaction est avantageusement au moins égale à la quantité nécessaire pour neutraliser l'acide halohydrique dégagé.

En d'autres termes, la quantité de base doit être suffisante pour assurer que le nucléophile soit toujours présent, au moins partiellement, à l'état libre (c'est-à-dire à l'état de vrai nucléophile) pendant la totalité de la réaction. Lorsque les bases sont oxydables, telles les amines, il préférable de limiter la coexistence de la base libre avec l'halogénure de sulfonyle.

Cela peut être réalisé, soit en limitant l'excès stoechiométrique par rapport aux acides qu'elles sont censées neutraliser à une fois, de préférence à une demi-fois, la QS, soit par une addition progressive de la base, ou les deux moyens à la fois. On peut également ajouter progressivement l'halogénure de sulfonyle.

Les techniques visées par la présente invention sont particulièrement bien adaptées à la sulfonylation par les chlorures d'alcoylsufonyle perfluorés, notamment pérfluorés sur le carbone porté par le soufre.

Le chlorure le plus utilisable est le chlorure de triflyle (CF₃SO₂Cl). D'une manière plus générale, la partie organique du chlorure de sulfonyle répond à la formule (Rf).

Par Rf on entend un radical de formule :

GEA -(CX₂)ₚ-

- où les X, semblables ou différents, représentent un chlore, un fluor ou un radical de formule CₙF₂ₙ₊₁ avec n entier au plus égal à 5 de préférence à 2, avec la condition qu'au moins un des X soit fluor, fluor avantageusement porté par le carbone relié au soufre ;
- où p représente un entier au plus égal à 2 ;
- où GEA représente un groupe électro-attracteur (c'est à dire sigma p supérieur à zéro, avantageusement à 0,1, de préférence à 0,2) dont les éventuelles fonctions sont inertes dans les conditions de la réaction, avantageusement fluor ou un reste perfluoré de formule CₙF₂ₙ₊₁ avec n entier au plus égal à 8, avantageusement à 5.

Le nombre total de carbone de Rf est avantageusement compris entre 1 et 15, de préférence entre 1 et 10.

La présente invention vise aussi un réactif utile pour la mise en oeuvre du procédé selon l'invention. Ce réactif comporte pour addition successive ou simultanée :
- un halogénure lourd c'est à dire, dont le nombre atomique est au moins égal à celui du chlore de sulfonyle, avantageusement chlorure de sulfonyle et
- une base organique comportant un atome d'azote trisubstitué et présentant un enchainement de formule > N-[C = C]_{D} C = N⁻, avec n égal à zéro ou un entier choisi dans l'intervalle fermé (c'est-à-dire comprenant les bornes) 1 à 4, avantageusement de 1 à 3, de préférence de 1 à 2
et par le fait que la partie organique dudit sulfonyle est perhalogénée, avantageusement perfluorée, sur le carbone porté par le soufre,

Ledit azote trisubstitué staturé est un trivalent dont le doublet est conjugué directement ou indirectement à une liaison π reliant deux atomes dont un au moins est un atome de la colonne VB.

Le réactif selon la présente invention peut, en outre, comporter, également pour addition successive ou simultanée, une base organique distincte de ladite base organique comportant un azote trisubstitué. En général, dans ce cas le rapport en équivalent entre la base organique comportant un azote trisubstitué (numérateur) et ladite base distincte (dénominateur) est compris dans l'intervalle fermé 1 pour mille 1/5, avantageusement entre 0,5% et 1/10.

Le réactif selon la présente invention peut, en outre, comporter, également pour addition successive ou simultanée, un solvant. Ce solvant, y compris le mélange de solvants, est avantageusement peu polaire et choisi parmi ceux présentant une faible solubilité dans l'eau. Dans certains cas, les chaînes aliphatiques chlorées ne sont pas satisfaisantes.

Comme pierre de touche de la polarité, on peut préciser que ledit solvant peu polaire est choisi parmi ceux, ou les mélanges de ceux, dont la polarité (Eₜ^{f} exprimée en kcal/mol) est au plus égale à 40, (avantageusement avec deux chiffres significatifs).

Ces solvants peu polaires sont usuellement choisis parmi les composés organiques oxygénés, notamment les éthers, les esters, voire les cétones, les hydrocarbures, y compris les fractions pétrolières, les hydrocarbures aromatiques halogénés sur le moyen. Ces derniers sont particulièrement intéressants, notamment les benzènes substitués et les hydrocarbures halogénés sur le noyau.

Ce qui vient d'être exposé précédemment convient particulièrement aux anilines, notamment celles dont ledit atome d'azote est relié à un aromatique à 6 chaînons, de préférence homocyclique, de préférence benzénique non condensé, lequel est avantageusement appauvri en électrons comme cela a déjà été précisé ci-dessus. Globalement, compte tenu des éventuels substituants particulièrement bien adaptés aux amines reliées à un aryle dont la richesse en électrons est au plus égale à un parachlorophényle (richesse évaluée au moyen des constantes sigma p de Hammett).

Parmi les groupes électro-attracteurs les plus usités, on peut citer les halogènes (chlore et fluor), les esters (de type CO-OR), les cétones, les amides non susceptibles d'interférer avec la sulfonylation, les alcoyles perhalogénés sur le carbone lié directement sur le noyau, notamment les alcoyles perfluorés sur l'atome proche du noyau, les nitriles, les groupes présentant une fonction sulfone ou phosphone directement liée au noyau.

La technique vise surtout la sulfonylation des anilines lato sensu (c'est-à-dire des amines portées par un aryle), mais on a également transposé cet enseignement à la sulfonamidation de l'ammoniac (-que) et la sulfimidation des sulfamides.

Dans ce cas, ces derniers sont avantageusement, sous la forme d'un sel de base organique, non alcoylables (c'est la base qui est non alcoylable).

Dans ces conditions, l'azote est porteur d'un hydrogène ou plus préférentiellement d'une charge négative (anion), alors que dans le cas des anilines qui a été visé ci-dessus, la fonction aniline comporte au moins un hydrogène, de préférence deux, pour des raisons d'encombrement stérique.

L'utilisation de ladite base organique comportant un atome métalloïde saturé, présentant une résonance avec une liaison π permet de réaliser avec un bon rendement la double sulfonylation des nucléophiles deux fois substituables (tels que l'ammoniac et les amines primaires). Les conditions opératoires préférées sont celles décrites dans la demande de brevet internationale publiée par l'OMPI sous le n°WO 98/52886, en utilisant au moins partiellement comme base ladite base organique comportant un atome métalloïde saturé, présentant une résonance avec une liaison π.

Pour des raisons d'hygiène du travail, les dérivés chlorés aliphatiques sont en général à éviter, bien qu'ils constituent une famille de solvants donnant de bons résultats, bien qu'elle ne soit pas la famille la plus performante, car leur solubilité, et notamment celle du chlorure de méthylène, est de l'ordre de 2% en volume, soit 2,6% en poids.

En ce qui concerne les solvants, il convient d'éviter, autant que faire se peut, les solvants présentant un caractère réducteur.

La réaction peut être menée de - 20°C à environ 200°C, plus généralement de 0° à environ 100°C.

Il est plus aisé de travailler à température et pression ambiantes mais on peut aussi être sous des pressions différentes, plus élevées. On peut également travailler dans une enceinte fermée (telle que autoclave ou tube scellé) et sous pression autogène.

Les exemples non limitatifs suivants illustrent l'invention.

### EXEMPLES

### Préparation du acétamido-5-diméthyl-2,4-trifluorométhanesulfonanilide

### - Réaction en présence de catalyseur :

Dans un réacteur, on charge successivement 350 g de dichlorométhane, 50,1 g de amino-5-diméthyl-2,4 acétanilide et 6,9 g de diméthylamino-4-pyridine. La suspension obtenue est ensuite agitée et refroidie à 10°C. On ajoute ensuite 57,4 g de triéthylamine 15 mn à 10°C.

Une solution de 56,8 g de chlorure de trifluorométhanesulfonyle dans 59 g de dichlorométhane est ensuite coulée en 2 h en maintenant la température du milieu à 10°C.

Le milieu est ensuite maintenu 2 h sous agitation à 10°C, puis on laisse remonter la température à 20°C et l'agitation est poursuivie pendant 2 h.

Le milieu est acidifié dans le réacteur par addition de 265 g d'une solution aqueuse de HCl à 4,2%.

Le précipité formé est ensuite filtré et rincé (3 fois) avec de l'eau permutée puis avec du dichlorométhane. Il est ensuite séché à 95°C sous pression réduite.

On obtient ainsi 67 g d'un solide beige, soit un rendement isolé de 77%.

Caractéristique du composé : Fusion = 180°C.

### - Réaction en l'absence de catalyseur :

Les conditions précédentes sont reprises mais en l'absence de 4-diméthylaminopyridine.

L'analyse du milieu réactionnel montre une dégradation de 90% du chlorure de trifluorométhanesulfonyle.

Rendement en produit attendu très inférieur à 10%.

### Préparation du chloro-5-(N-trifluorométhylsulfonyl)amino-2 benzoate de méthyle

### - Réaction en présence de catalyseur :

Dans un réacteur, on charge successivement 17 g de chloro-5 anthranilate de méthyle, 120 g de dichlorométhane, 2,26 g de diméthylamino-4 pyridine, puis 18,5 g de chlorure de trifluorométhanesulfonyle. La solution est agitée à 15°C et on coule en 3 h une solution de 18,7 g de triéthylamine dans 19 g de dichlorométhane. Après addition, le milieu est agité 3 h à 15°C, puis 10 h à la température ambiante.

Le milieu est ensuite lavé successivement avec 150 g d'eau permutée, avec 100 g d'une solution d'acide chlorhydrique concentré, puis à nouveau avec 160 g d'eau permutée.

Le solvant est éliminé sous pression réduite.

On obtient ainsi 27,7 g d'un solide jaunâtre, soit un rendement brut isolé de 95%.

Caractéristique du composé : Fusion = 81°C.

### - Réaction en l'absence de catalyseur :

### 1/ Réaction avec de la triéthylamine :

Les conditions précédentes sont reprises mais en l'absence de 4-diméthylaminopyridine.

L'analyse du milieu réactionnel montre une dégradation totale du chlorure de trifluorométhanesulfonyle.

Le produit attendu n'a pas été formé.

### 2/ Réaction avec de la diisopropyléthylamine :

De la même manière, l'essai en l'absence de catalyseur et en présence de diisopropyléthylamine ne conduit pas au produit attendu.

### 3/ Réaction avec 1,4-diazabicyclo[2.2.2]octane ou DABCO :

A nouveau le produit attendu n'est pas formé.

### Préparation du bis trifluorométhanesulfonimide de trialcoyl ammonium

### - Réaction catalysée:

### Exemple 1

Dans un réacteur, on charge 9,3 g de triéthylamine (0,092 M), 0,56 g de 4-diméthyl aminopyridine et 45 g de dichlorométhane. On ajoute ensuite 1,38 g (0,092 M) d'ammoniac. La solution obtenue est agitée à 0°C.

Le chlorure de trifluorométhanesulfonyle (15,5 g, soit 0092 M) dilué dans 10 ml de dichlorométhane est additionné ensuite en 2 h à 0°C, puis la réaction est poursuivie 3 h à température ambiante.

L'analyse du milieu réactionnel montre un taux d'impureté trifluorométhanesulfinate de 2%.

Le milieu réactionnel est traité 2 fois par 15 ml d'une solution aqueuse HCl 12%, puis à 3 reprises par 15 ml d'eau. La phase organique résiduelle est concentrée sous pression réduite, on obtient alors 13,65 g de bis trifluorométhanesulfonimide de triéthylammonium qui se présente sous forme de liquide, soit un rendement isolé de 80%.

La pureté du composé obtenu est de 98%.

### Exemple 2

Le mode opératoire de l'exemple 1 est repris mais en remplaçant le dichlorométhane par du dioxanne. Le milieu réactionnel est analysé après dilution à l'eau : le rendement en bis trifluorométhanesulfonimide en fin de réaction est de 51%, avec un taux de trifluorométhanesulfinate de 6%.

### - Réaction non catalysée :

### Exemple 3 comparatif

Le mode opératoire de l'exemple 1 est reconduit mais sans utilisation de 4-diméthyl aminopyridine.

Le rendement en bis trifluorométhanesulfonimide en fin de réaction est de 47%, avec un taux de trifluorométhanesulfinate de 21%.

### Exemple 4 comparatif

Le mode opératoire 3 est reconduit mais en utilisant la diisopropyl éthylamine comme base en remplacement de la triéthylamine.

Le rendement en bis trifluorométhanesulfonimide en fin de réaction est de 51%, avec un taux de trifluorométhanesulfinate de 21%.

### Exemple 5 comparatif

Dans le réacteur, on charge 11,8 g de chlorure de trifluorométhanesulfonyle (0,07 M) et on ajoute ensuite à 0°C 33,9 g de triéthylamine (0,0336 M) en 10 mn.

Le milieu est agité 10 mn encore à 0°C puis on ajoute 0,5 g (0,029 M) d'ammoniac. La réaction est exothermique. Le milieu est ensuite porté à 65°C pendant 4 h.

On observe des composés issus de la dégradation oxydante des amines. Le produit majoritaire est le trifluorométhanesulfinate avec un taux de 57%.

La présence de bis trifluorométhanesulfonimide n'est pas mise en évidence, ce qui implique un rendement de réaction inférieur à 2%, voire nul.

## Revendications

1. Procédé de sulfonylation, **caractérisé par le fait qu'**il comprend une étape de mise en contact d'un nucléophile dont l'atome nucléophile est avantageusement un azote avec un réactif comportant pour addition successive ou simultanée :
• un halogénure lourd c'est à dire, dont le nombre atomique est au moins égal à celui du chlore de sulfonyle avantageusement chlorure de sulfonyle
et
• une base organique comportant un azote trisubstitué et présentant un enchaînement de formule > N-[C = C]ₙ-C = N- avec n égal à zéro ou un entier choisi dans l'intervalle fermé 1 à 4, avantageusement de 1 à 3, de préférence de 1 à 2, et **par le fait que** la partie organique dudit sulfonyle est perhalogénée, avantageusement perfluorée, sur le carbone porté par le soufre.

2. Procédé selon la revendication 1, **caractérisé par le fait que** l'acide associé dudit nucléophile présente un pKa au plus égal à environ 7, avantageusement à 6, de préférence à 5.

3. Procédé selon les revendications 1 et 2, **caractérisé par le fait que** ledit azote dudit nucléophile est relié à un groupe électroattracteur.

4. Procédé selon la revendication 3, **caractérisé par le fait que** ledit groupe électro-attracteur est choisi parmi les aryles avantageusement appauvris en électrons et les sulfonyles.

5. Procédé selon les revendications 1 à 4, **caractérisé par le fait que** ladite base organique comportant un atome trivalent de la colonne VB (colonne de l'azote) dont le doublet est conjugué, directement ou indirectement, à une liaison π comporte un enchaînement de formule >N-[C(R₁) = C(R₂)]n-C(R₃) = N-, avec n égal à zéro ou un entier choisi dans l'intervalle fermé (c'est-à-dire comprenant les bornes) 1 à 4, avantageusement de 1 à 3, de préférence de 1 à 2 et où R₁, R₂ et R₃, semblables ou différents, sont choisis parmi les dérivés hydrocarbonés, avantageusement alcoyles d'au plus 4 atomes de carbone et l'hydrogène.

6. Procédé selon les revendications 1 à 5, **caractérisé par le fait que** ledit azote trisubstitué est une amine tertiaire (forme une amine tertiaire).

7. Procédé selon les revendications 1 à 6, **caractérisé par le fait que** ladite base organique comporte un enchaînement de formule :
(R₅)(R₄)N-[C(R₁) = C(R₂)]ₙ-C=N-R₆
avec n égal à zéro ou un entier choisi dans l'intervalle fermé (c'est-à-dire comprenant les bornes) 1 à 4, avantageusement de 1 à 3, de préférence de 1 à 2 et où R₁, R₂ et R₆, semblables ou différents, sont choisis parmi les groupes hydrocarbonés, avantageusement alcoyles d'au plus 4 atomes de carbone et l'hydrogène et où R₄ et R₅, semblables ou différents, sont choisis parmi les groupes hydrocarbonés, avantageusement alcoyles d'au plus 4 atomes de carbone, un ou deux des substituants R₁, R₂, R₃, R₄, R₅ et R₆ pouvant être reliés à d'autre(s) substituant(s) restant pour former un-ou plusieurs cycles.

8. Procédé selon les revendications 1 à 7, **caractérisé par le fait que** ladite liaison π reliant deux atomes est intracyclique.

9. Procédé selon les revendications 1 à 8, **caractérisé par le fait que** ladite liaison π reliant deux atomes est intracyclique d'un cycle aromatique.

10. Procédé selon la revendication 9, **caractérisé par le fait que** ledit cycle est un cycle pyridinique.

11. Procédé selon les revendications 1 à 10, **caractérisé par le fait que** ladite base est choisie parmi les para- ou ortho-dialcoylaminopyridines.

12. Procédé selon les revendications 1 à 11, **caractérisé par le fait que** ledit nucléophile présente comme atome nucléophile un azote.

13. Procédé selon les revendications 1 à 12, **caractérisé par le fait que** ledit nucléophile est choisi parmi l'ammoniac et/ou un sulfonamide.

14. Procédé selon les revendications 1 à 13, **caractérisé par le fait que** ledit nucléophile est l'ammoniac et **par le fait qu'** il est perfluorosulfoné deux fois.

15. Procédé selon les revendications 1 à 14, **caractérisé par le fait que** ledit nucléophile est une arylamine dans laquelle les substituants, hors la fonction nucléophile, du noyau porteur de l'atome nucléophile sont tels que la somme de leurs constantes de Hammett σₚ soit au moins égale à 0,14, avantageusement à 0,20.

16. Procédé selon les revendications 1 à 15, **caractérisé par le fait que** ledit réactif comporte en outre, pour addition successive ou simultanée, une base organique non alcoylable.

17. Procédé selon la revendication 16, **caractérisé par le fait que** ladite base organique non alcoylable est choisie parmi les dialcoylphosphines encombrées, les trialcoylphosphines, les hydroxydes de phosphonium, les dialcoylamines encombrées, les trialcoylamiaes, les hydroxydes d'ammonium.

18. Procédé selon les revendications 1 à 17, **caractérisé par le fait que** ladite mise en contact est réalisée dans un solvant organique, avantageusement peu polaire, de préférence peu miscible à l'eau (au plus 10% en masse, avantageusement au plus 5% en masse, de préférence au plus 2% en masse).

19. Procédé selon les revendications 1 à 18, **caractérisé par le fait que** la quantité de ladite base non alcoylable et liposoluble mise en jeu au cours de la réaction est au moins égale à la quantité nécessaire pour neutraliser l'acide halohydrique dégagé.

20. Procédé selon les revendications 1 à 19, **caractérisé par le fait que** la partie organique dudit chlorure de sulfonyle est perfluorée sur le carbone porté par le soufre.

21. Procédé selon les revendications 1 à 20, **caractérisé par le fait que** la partie organique dudit chlorure de sulfonyle est choisie parmi les radicaux de formule (Rf) :
GEA -(CX₂)ₚ-
- où les X, semblables ou différents, représentent un chlore, un fluor ou un radical de formule CₙF₂ₙ₊₁, avec n entier au plus égal à 5, de préférence à 2, avec la condition qu'au moins un des X soit fluor;
- où p représente un entier au plus égal à 2 ;
- où GEA représente un groupe électioattracteur dont les éventuelles fonctions sont inertes dans les conditions de la réaction, avantageusement fluor ou un reste perfluoré de formule CₙF₂ₙ₊₁, avec n entier au plus égal à 8, avantageusement à 5, le nombre total de carbone de Rf étant avantageusement compris entre 1 et 15, de préférence entre 1 et 10.

22. Réactif utile pour la mise en oeuvre du procédé selon les revendications 1 à 21, **caractérisé par le fait qu'**il comporte pour addition successive ou simultanée :
• un halogénure lourd c'est à dire, dont le nombre atomique est au moins égal à celui du chlore de sulfonyle, avantageusement chlorure de sulfonyle
• une base organique comportant un atome d'azote trisubstitué et présentant un enchaînement de formule > N-[C = C]ₙ⁻C = N⁻, avec n égal à zéro ou un entier choisi dans l'intervalle fermé (c'est-à-dire comprenant les bornes) 1 à 4, avantageusement de 1 à 3, de préférence de 1 à 2.
et **par le fait que** la partie organique dudit sulfonyle est perhalogénée, avantageusement perfluorée, sur le carbone porté par le soufre,

23. Réactif selon la revendication 22, **caractérisé par le fait qu'**il comporte en outre, pour addition successive ou simultanée, un solvant, ledit solvant, y compris mélange de solvant, étant peu polaire et choisi parmi ceux présentant une faible solubilité dans l'eau, et avantageusement ne présentant pas une chaîne aliphatique chlorée.

24. Réactif selon la revendication 23, **caractérisé par le fait que** ledit solvant peu polaire est choisi parmi ceux dont la polarité (E^{f}ₜ exprimée en kcal par mol) est au plus égale à 40 (avantageusement deux chiffres significatifs).

25. Réactif selon les revendications 23 et 24, **caractérisé par le fait que** ledit solvant peu polaire est choisi parmi les composés organiques oxygénés, (notamment les éthers, les esters, voire les cétones), les hydrocarbures (y compris fractions pétrolières), les hydrocarbures aromatiques halogènes sur le noyau.

26. Réactif selon les revendications 23 à 25, **caractérisé par le fait que** ledit solvant peu polaire est choisi parmi les benzènes substitués et les hydrocarbures halogénés sur le noyau.

## Patentansprüche

1. Verfahren zur Sulfonylierung, **dadurch gekennzeichnet, daß** es eine Stufe zum In-Kontakt-Bringen von einem Nucleophil, dessen nucleophiles Atom vorteilhafterweise ein Stickstoff ist, mit einem Reaktanden einschließt, der für die aufeinanderfolgende oder gleichzeitige Addition umfaßt:
· ein schweres Halogenid, das heißt, daß seine Atomnummer mindestens gleich der von Chlor ist, von Sulfonyl, vorteilhafterweise Sulfonylchlorid, und
· eine organische Base, umfassend einen trisubstituierten Stickstoff, der eine Verkettung der Formel >N-[C=C]-C=N- mit n gleich null oder einem Ganzen, gewählt aus dem geschlossenen Intervall von 1 bis 4, vorteilhafterweise von 1 bis 3, vorzugsweise von 1 bis 2, aufweist,
und **dadurch**, daß der organische Teil des genannten Sulfonyls an dem Kohlenstoff, der vom Schwefel getragen wird, perhalogeniert, vorteilhafterweise perfluoriert ist.

2. Verfahren nach Anspruch 1, **dadurch gekennzeichnet, daß** die assoziierte Säure des genannten Nucleophils einen pKa von höchstens gleich 7, vorteilhafterweise 6, vorzugsweise 5 aufweist.

3. Verfahren nach Anspruch 1 und 2, **dadurch gekennzeichnet, daß** der genannte Stickstoff des genannten Nucleophils mit einer elektroattraktiven Gruppe verbunden ist.

4. Verfahren nach Anspruch 3, **dadurch gekennzeichnet, daß** die genannte elektroattraktive Gruppe unter den Arylen, vorteilhafterweise an Elektronen verarmt, und den Sulfonylen ausgewählt wird.

5. Verfahren nach Anspruch 1 bis 4, **dadurch gekennzeichnet, daß** die genannte organische Base, die ein trivalentes Atom aus der Reihe VB (Stickstoffreihe) umfaßt, dessen Dublett, direkt oder indirekt, mit einer Bindung π konjugiert ist, eine Verkettung der Formel >N-[C(R₁)=C(R₂)]ₙ-C(R₃)=N- mit n gleich null oder einem Ganzen, gewählt aus dem geschlossenen Intervall (das heißt, einschließlich der Grenzwerte) von 1 bis 4, vorteilhafterweise von 1 bis 3, vorzugsweise von 1 bis 2, umfaßt, und worin R₁, R₂ und R₃, gleich oder verschieden, unter den Kohlenwasserstoff-Derivaten, vorteilhafterweise Alkyl mit höchstens 4 Kohlenstoffatomen, und Wasserstoff ausgewählt werden.

6. Verfahren nach Anspruch 1 bis 5, **dadurch gekennzeichnet, daß** der genannte trisubstituierte Stickstoff ein tertiäres Amin (Form eines tertiären Amins) ist.

7. Verfahren nach Anspruch 1 bis 6, **dadurch gekennzeichnet, daß** die genannte organische Base eine Verkettung der Formel
(R₅) (R₄)N-[C(R₁)=C(R₂)]ₙ-C=N-R₆
mit n gleich null oder einem Ganzen, gewählt aus dem geschlossenen Intervall (das heißt, einschließlich der Grenzwerte) von 1 bis 4, vorteilhafterweise von 1 bis 3, vorzugsweise von 1 bis 2, umfaßt, und worin R₁, R₂ und R₆, gleich oder verschieden, unter den Kohlenwasserstoff-Gruppen, vorteilhafterweise Alkyl mit höchstens 4 Kohlenstoffatomen, und Wasserstoff, und worin R₄ und R₅, gleich oder verschieden, unter den Kohlenwasserstoff-Gruppen, vorteilhafterweise Alkyl mit höchstens 4 Kohlenstoffatomen, ausgewählt werden, wobei einer oder zwei der Substituenten R₁, R₂, R₃, R₄, R₅ und R₆, der (die) mit anderem(n) Substituent(en) verbunden sein kann (können) verbleiben, um einen oder mehrere Ringe zu bilden.

8. Verfahren nach Anspruch 1 bis 7, **dadurch gekennzeichnet, daß** die genannte Bindung π, die zwei Atome verbindet, intracyclisch ist.

9. Verfahren nach Anspruch 1 bis 8, **dadurch gekennzeichnet, daß** die genannte Bindung n, die zwei Atome verbindet, intracyclisch von einem aromatischen Ring ist.

10. Verfahren nach Anspruch 1 bis 9, **dadurch gekennzeichnet, daß** der genannte Ring ein Pyridin-Ring ist.

11. Verfahren nach Anspruch 1 bis 10, **dadurch gekennzeichnet, daß** die genannte Base unter den para- oder ortho-Dialkylaminopyridinen ausgewählt wird.

12. Verfahren nach Anspruch 1 bis 11, **dadurch gekennzeichnet, daß** das genannte Nucleophil als nucleophiles Atom einen Stickstoff aufweist.

13. Verfahren nach Anspruch 1 bis 12, **dadurch gekennzeichnet, daß** das genannte Nucleophil unter Ammoniak und/oder einem Sulfonamid ausgewählt wird.

14. Verfahren nach Anspruch 1 bis 13, **dadurch gekennzeichnet, daß** das genannte Nucleophil Ammoniak ist, und **dadurch**, daß es zweimal perfluorsulfoniert ist.

15. Verfahren nach Anspruch 1 bis 14, **dadurch gekennzeichnet, daß** das genannte Nucleophil ein Arylamin ist, bei dem die Substituenten außer der nucleophilen Funktion des Kerns, der das nucleophile Atom trägt, derart sind, daß die Summe ihrer Hammett-Konstanten σₚ mindestens gleich 0,14, vorteilhafterweise 0,20 ist.

16. Verfahren nach Anspruch 1 bis 15, **dadurch gekennzeichnet, daß** der genannte Reaktand außerdem für eine aufeinanderfolgende oder gleichzeitige Addition eine nicht alkylierbare organische Base umfaßt.

17. Verfahren nach Anspruch 16, **dadurch gekennzeichnet, daß** die nicht alkylierbare organische Base unter den sterisch sperrigen Dialkylphosphinen, den Trialkylphosphinen, den Phosphoniumhydroxiden, den sterisch sperrigen Dialkylaminen, den Trialkylaminen und den Ammoniumhydroxiden ausgewählt wird.

18. Verfahren nach Anspruch 1 bis 17, **dadurch gekennzeichnet, daß** das genannte In-Kontakt-Bringen in einem organischen Lösungsmittel realisiert wird, das vorteilhafterweise wenig polar und vorzugsweise wenig mit Wasser mischbar ist (höchstens 10 Masse-%, vorteilhafterweise höchstens 5 Masse-%, vorzugsweise höchstens 2 Masse-%).

19. Verfahren nach Anspruch 1 bis 18, **dadurch gekennzeichnet, daß** die Menge der genannten, im Verlauf der Reaktion eingesetzten, nicht alkylierbaren und fettlöslichen Base mindestens gleich der Menge ist, die für die Neutralisierung der freiwerdenden Halogenwasserstoffsäure notwendig ist.

20. Verfahren nach Anspruch 1 bis 19, **dadurch gekennzeichnet, daß** der organische Teil des genannten Sulfonylchlorids an dem Kohlenstoff, der von dem Schwefel getragen wird, perfluoriert ist.

21. Verfahren nach Anspruch 1 bis 20, **dadurch gekennzeichnet, daß** der organische Teil des genannten Sulfonylchlorids unter den Resten der Formel (Rf)
GEA-(CX₂)ₚ-
ausgewählt wird,
- worin die X, gleich oder verschieden, ein Chlor, ein Fluor oder einen Rest der Formel CₙF₂ₙ₊₁ mit n einem Ganzen von höchstens gleich 5, vorzugsweise 2 darstellen, unter der Bedingung, daß mindestens eines der X Fluor ist;
- worin p ein Ganzes von höchstens gleich 2 ist;
- worin GEA eine elektroattraktive Gruppe, deren eventuelle Funktionen unter den Bedingungen der Reaktion inert sind, darstellt, vorzugsweise Fluor oder einen perfluorierten Rest der Formel CₙF₂ₙ₊₁ mit n einem Ganzen von höchstens gleich 8, vorteilhafterweise 5, wobei die Gesamtzahl der Kohlenstoffe von Rf vorteilhafterweise zwischen einschließlich 1 und 15, vorzugsweise zwischen 1 und 10 beträgt.

22. Reaktand, geeignet für die Durchführung des Verfahrens nach Anspruch 1 bis 21, **dadurch gekennzeichnet, daß** er für die aufeinanderfolgende oder gleichzeitige Addition umfaßt:
· ein schweres Halogenid, das heißt, daß seine Atomnummer mindestens gleich der von Chlor ist, von Sulfonyl, vorteilhafterweise Sulfonylchlorid, und
· eine organische Base, umfassend einen trisubstituierten Stickstoff, der eine Verkettung der Formel >N-[C=C]ₙ-C=N- mit n gleich null oder einem Ganzen, gewählt aus dem geschlossenen Intervall (das heißt, einschließlich der Grenzwerte) von 1 bis 4, vorteilhafterweise von 1 bis 3, vorzugsweise von 1 bis 2, aufweist,
und **dadurch**, daß der organische Teil des genannten Sulfonyls an dem Kohlenstoff, der vom Schwefel getragen wird, perhalogeniert, vorteilhafterweise perfluoriert ist.

23. Reaktand nach Anspruch 22, **dadurch gekennzeichnet, daß** er außerdem für die aufeinanderfolgende oder gleichzeitige Addition ein Lösungsmittel umfaßt, wobei das genannte Lösungsmittel einschließlich der Lösungsmittelmischung wenig polar ist und ausgewählt wird unter denjenigen, die eine geringe Löslichkeit in Wasser aufweisen, und vorteilhafterweise unter denjenigen, die keine chlorierte aliphatische Kette besitzen.

24. Reaktand nach Anspruch 23, **dadurch gekennzeichnet, daß** das genannte Lösungsmittel wenig polar ist und ausgewählt wird unter denjenigen, deren Polarität (E^{f}ₜ ausgedrückt in kcal pro mol) höchstens gleich 40 (vorteilhafterweise zwei signifikante Ziffern) beträgt.

25. Reaktand nach Anspruch 23 und 24, **dadurch gekennzeichnet, daß** das genannte Lösungsmittel wenig polar ist und ausgewählt wird unter den organischen Sauerstoff-Verbindungen (insbesondere den Ethern, den Estern, sogar den Ketonen), den Kohlenwasserstoffen (einschließlich Erdölfraktionen) und den am Kern halogenierten aromatischen Kohlenwasserstoffen.

26. Reaktand nach Anspruch 23 bis 25, **dadurch gekennzeichnet, daß** das genannte Lösungsmittel wenig polar ist und ausgewählt wird unter den substituierten Benzolen und den am Kern halogenierten Kohlenwasserstoffen.

## Claims

1. Sulphonylation process, **characterized in that** it comprises a step of placing a nucleophile, whose nucleophilic atom is advantageously a nitrogen, in contact with a reagent comprising, for successive or simultaneous addition:
- a heavy halide (i.e. a halide whose atomic number is at least equal to that of chlorine) of sulphonyl, advantageously sulphonyl chloride, and;
- an organic base comprising a trisubstituted nitrogen and having a sequence of formula > N-[C = C]ₙ-C = N- with n = zero or an integer chosen in the closed range 1 to 4, advantageously from 1 to 3, preferably from 1 to 2, and **in that** the organic part of the said sulphonyl is perhalogenated, advantageously perfluorinated, on the carbon borne by the sulphur.

2. Process according to claim 1, **characterized in that** the conjugate acid of the said nucleophile has a pKa of not more than about 7, advantageously not more than 6 and preferably not more than 5.

3. Process according to claims 1 and 2, **characterized in that** the said nitrogen of the said nucleophile is linked to an electron-withdrawing group.

4. Process according to claim 3, **characterized in that** the said electron-withdrawing group is chosen from aryls, advantageously electron-depleted aryls, and sulphonyls.

5. Process according to claims 1 to 4, **characterized in that** the said organic base comprising a trivalent atom from column VB (the nitrogen column), whose lone pair is conjugated directly or indirectly to a π bond, comprises a sequence of formula >N-[C(R₁) = C(R₂)]ₙ-C(R₃) = N- with n = zero or an integer chosen in the closed range (i.e. comprising the limits) 1 to 4, advantageously from 1 to 3, preferably from 1 to 2, and in which R₁, R₂ and R₃, which may be identical or different, are chosen from hydrocarbon-based derivatives, advantageously alkyl derivatives containing not more than 4 carbon atoms, and hydrogen.

6. Process according to claims 1 to 5, **characterized in that** the said trisubstituted nitrogen is a tertiary amine (forms a tertiary amine).

7. Process according to claims 1 to 6, **characterized in that** the said organic base comprises a sequence of formula
(R₅)(R₄)N-[C(R₁) = C(R₂)]ₙ-C = N-R₆
with n = 0 or an integer chosen in the closed range (i.e. comprising the limits) 1 to 4, advantageously from 1 to 3, preferably from 1 to 2, and in which R₁, R₂ and R₆, which may be identical or different, are chosen from hydrocarbon-based groups, advantageously alkyl groups containing not more than 4 carbon atoms, and hydrogen, and in which R₄ and R₅, which may be identical or different, are chosen from hydrocarbon-based groups, advantageously alkyl groups containing not more than 4 carbon atoms, one or two of the substituents R₁, R₂, R₃, R₄, R₅ and R₆ being able to be linked to other substituent(s) remaining to form one or more rings.

8. Process according to claims 1 to 7, **characterized in that** the said n bond linking two atoms is endocyclic.

9. Process according to claims 1 to 8, **characterized in that** the said π bond linking two atoms is endocyclic in an aromatic ring.

10. Process according to claim 9, **characterized in that** the said ring is a pyridine ring.

11. Process according to claims 1 to 10, **characterized in that** the said base is chosen from para- or ortho-dialkylaminopyridines.

12. Process according to claims 1 to 11, **characterized in that** the said nucleophile has a nitrogen as nucleophilic atom.

13. Process according to claims 1 to 12, **characterized in that** the said nucleophile is chosen from ammonia and/or a sulphonamide.

14. Process according to claims 1 to 13, **characterized in that** the said nucleophile is ammonia and **in that** it is perfluorosulphonated twice.

15. Process according to claims 1 to 14, **characterized in that** the said nucleophile is an arylamine in which the substituents, excluding the nucleophilic function, of the ring bearing the nucleophilic atom are such that the sum of their Hammett σₚ constants is at least equal to 0.14, advantageously to 0.20.

16. Process according to claims 1 to 15, **characterized in that** the said reagent also comprises, for successive or simultaneous addition, a non-alkylatable organic base.

17. Process according to claim 16, **characterized in that** the said non-alkylatable organic base is chosen from bulky dialkylphosphines, trialkylphosphines, phosphonium hydroxides, bulky dialkylamines, trialkylamines and ammonium hydroxides.

18. Process according to claims 1 to 17, **characterized in that** the said placing in contact is carried out in an organic solvent which is advantageously relatively non-polar and preferably relatively immiscible with water (not more than 10% by mass, advantageously not more than 5% by mass and preferably not more than 2% by mass)

19. Process according to claims 1 to 18, **characterized in that** the amount of the said non-alkylatable liposoluble base used during the reaction is at least equal to the amount required to neutralize the hydrohalic acid released.

20. Process according to claims 1 to 19, **characterized in that** the organic part of the said sulphonyl chloride is perfluorinated on the carbon borne by the sulphur.

21. Process according to claims 1 to 20, **characterized in that** the organic part of the said sulphonyl chloride is chosen from the radicals of formula (Rf):
EWG-(CX₂)ₚ-
- in which the groups X, which may be identical or different, represent a chlorine, a fluorine or a radical of formula CₙF₂ₙ₊₁ where n is an integer of not more than 5, preferably not more than 2, with the condition that at least one of the groups X is fluorine;
- in which p represents an integer of not more than 2;
- in which EWG represents an electron-withdrawing group whose possible functions are inert under the reaction conditions, advantageously fluorine or a perfluoro residue of formula CₙF₂ₙ₊₁ with n being an integer of not more than 8, advantageously not more than 5, the total number of carbons in Rf being advantageously between 1 and 15, preferably between 1 and 10.

22. Reagent which is useful for carrying out the process according to claims 1 to 21, **characterized in that** it comprises, for successive or simultaneous addition:
• a heavy halide (i.e. a halide whose atomic number is at least equal to that of chlorine) of sulphonyl, advantageously sulphonyl chloride, and:
• an organic base comprising a trisubstituted nitrogen atom and having a sequence of formula >N-[C=C]ₙ⁻C=N⁻ with n = zero or an integer chosen in the closed range (i.e. comprising the limits) 1 to 4, advantageously from 1 to 3, preferably from 1 to 2, and **in that** the organic part of the said sulphonyl is perhalogenated, advantageously perfluorinated, on the carbon borne by the sulphur.

23. Reagent according to claim 22, **characterized in that** it also comprises, for successive or simultaneous addition, a solvent, the said solvent, including a solvent mixture, being relatively non-polar and chosen from those which have low solubility in water, and advantageously which do not contain a chlorinated aliphatic chain.

24. Reagent according to claim 23, **characterized in that** the said relatively non-polar solvent is chosen from those whose polarity (E^{f}ₜ expressed in kcal/mol) is not more than 40 (advantageously two significant figures).

25. Reagent according to claims 23 and 24, **characterized in that** the said relatively non-polar solvent is chosen from oxygenated organic compounds, (in particular ethers, esters or even ketones), hydrocarbons (including petroleum fractions) and aromatic hydrocarbons which are halogenated on the ring.

26. Reagent according to claims 23 to 25, **characterized in that** the said relatively non-polar solvent is chosen from substituted benzenes and hydrocarbons which are halogenated on the ring.
